(19) **Europäisches Patentamt**
**European Patent Office**
**Office européen des brevets**

(11) **EP 0 623 575 B1**

(12) **EUROPÄISCHE PATENTSCHRIFT**

(45) Veröffentlichungstag und Bekanntmachung des Hinweises auf die Patenterteilung:
**11.11.1998 Patentblatt 1998/46**

(51) Int. Cl.$^6$: **C07C 45/67**, C07C 49/167, C07C 67/10, C07C 69/63

(21) Anmeldenummer: 94106031.1

(22) Anmeldetag: 19.04.1994

(54) **Verfahren zur Herstellung von Ketonen**

Process for the preparation of ketones

Procédé pour la préparation de cétones

(84) Benannte Vertragsstaaten:
**AT BE CH DE DK ES FR GB GR IE IT LI LU NL PT**

(30) Priorität: 27.04.1993 DE 4313794
24.06.1993 DE 4321017

(43) Veröffentlichungstag der Anmeldung:
**09.11.1994 Patentblatt 1994/45**

(73) Patentinhaber:
• **Solvay Fluor und Derivate GmbH**
**D-30173 Hannover (DE)**
Benannte Vertragsstaaten:
**BE CH DE DK ES FR GB GR IE IT LI LU NL PT**
• **Braun, Max, Dr.**
**30900 Wedemark (DE)**
Benannte Vertragsstaaten:
**AT**

(72) Erfinder:
• **Braun, Max, Dr.**
**D-30900 Wedemark (DE)**
• **Eicher, Johannes, Dr.**
**D-30826 Garbsen (DE)**
• **Rudolph, Werner, Dr.**
**D-30559 Hannover (DE)**
• **Eichholz, Kerstin**
**D-30855 Langenhagen (DE)**

(74) Vertreter: **Lauer, Dieter, Dr.**
**Solvay Pharmaceuticals GmbH,**
**Hans-Böckler-Allee 20**
**30173 Hannover (DE)**

(56) Entgegenhaltungen:
**US-A- 2 369 250**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die Erfindung bezieht sich auf ein Verfahren zur Herstellung von Ketonen.

Ketone sind wertvolle Zwischenprodukte in der chemischen Synthese. 1.1.1-Trifluoraceton beispielsweise ist ein wichtiges Zwischenprodukt bei der Herstellung biologisch aktiver Zwischenverbindungen, siehe DE-OS 40 25 188. In dieser Offenlegungsschrift wird die Herstellung von Halogenmethylketonen aus der jeweiligen Halogenmethylnitro-Verbindung durch Umsetzung mit Alkoholat und anschließender Ozonisierung beschrieben. Das Produkt fällt als Hydrat, Acetal oder Halbacetal an. Die genannte Offenlegungsschrift beschreibt weitere Verfahren zur Ketonherstellung, die über Grignard-Verbindungen ablaufen (dieser Reaktionstyp macht erhöhte Sicherheitsvorkehrungen notwendig, außerdem werden unerwünschte Abfallprodukte produziert) oder die säurekatalysierte Spaltung von Ketoestern betreffen. Bei dem letzteren Verfahren fallen ebenfalls Hydrate der Ketone an. Ferner wird oft eine Deacylierung beobachtet statt der gewünschten Decarboxylierung. Andere Verfahren sehen eine Katalyse durch Übergangsmetalle vor; Ketone mit einer $CF_3$-Gruppe könnten durch Metalle komplexiert werden.

Die US-A 2,369,250 offenbart ein Verfahren zur Herstellung von β-Diketonen. Dabei wird der Ester einer β-Ketocarbonsäure mit einem Carbonsäureanhydrid unter Bildung des β-Diketons, eines Carbonsäureesters und $CO_2$ umgesetzt. Es wird als äußerst wünschenswert angesehen, die Reaktion in Anwesenheit eines Metalles der II. Hauptgruppe des Periodensystems der Elemente, Eisen oder Mangen durchzuführen.

Aufgabe der vorliegenden Erfindung ist es, ein Verfahren anzugeben, bei welchem unmittelbar wasserfreie Ketone ohne Dehydratisierung anfallen und das technisch einfach durchzuführen ist. Diese Aufgabe wird durch das Verfahren der vorliegenden Erfindung gelöst.

Das erfindungsgemäße Verfahren zur Herstellung von Ketonen aus Keto-Ester-Verbindungen sieht vor, daß man die Keto-Ester-Verbindung mit einer Carbonsäure umestert und die freigesetzte Säure decarboxyliert, um das Keton zu erhalten, und umfaßt die Herstellung von Ketonen der allgemeinen Formel (I)

$$R^1C(O)CH_nR^2{}_{3-n} \qquad\qquad (I)$$

worin $R^1$ Alkyl mit 1 bis 10 C-Atomen; durch mindestens 1 Halogenatom substituiertes Alkyl mit 1 bis 10 C-Atomen; Aryl; durch mindestens 1 Halogenatom substituiertes Aryl; Arylalkyl; $R^2$ für Wasserstoff; Alkyl mit 1 bis 10 C-Atomen; $C_1$-$C_{10}$-alkyl, welches durch mindestens 1 Halogenatom substituiert ist; Aryl; durch mindestens 1 Halogenatom substituiertes Aryl; Arylalkyl; Halogen oder $C(O)R^3$ steht, worin $R^3$ Alkyl mit 1 bis 10 C-Atomen; durch mindestens 1 Halogenatom substituiertes Alkyl mit 1 bis 10 C-Atomen; Aryl; durch mindestens 1 Halogenatom substituiertes Aryl; Arylalkyl bedeutet und n=1 oder 2 bedeutet, durch Umsetzung einer Carbonsäure mit 1 bis 10 C-Atomen oder einer durch mindestens 1 Halogenatom substituierten Carbonsäure mit 1 bis 10 C-Atomen bei einer Temperatur von mindestens 70°C mit einer Keto-Ester-Verbindung in Form einer Carboxylat- oder Dicarboxylatverbindung der allgemeinen Formel (II)

$$R^1C(O)CR^2{}_{3-n}[C(0)OR^4]_n \qquad\qquad (II)$$

worin n, $R^1$ und $R^2$ die obenbezeichnete Bedeutung besitzen und $R^4$ Alkyl mit 1 bis 10 C-Atomen; durch mindestens 1 Halogenatom substituiertes Alkyl mit 1 bis 10 C-Atomen; Arylalkyl; Aryl; durch mindestens 1 Halogenatom substituiertes Aryl bedeutet.

Der Begriff "freigesetzte Säure" bezeichnet die, mindestens eine Keto-Gruppe aufweisende Carbonsäure, die der eingesetzten Keto-Ester-Verbindung entspricht. Vorzugsweise setzt man β-Keto-Ester ein.

Im Unterschied zu bekannten Verfahren erfolgt beim erfindungsgemäßen Verfahren eine Umesterung; deshalb arbeitet man vorzugsweise ohne Zusatz von Wasser (beispielsweise ohne Zusatz wäßriger Säurelösungen). Besonders bevorzugt ist das Arbeiten in Abwesenheit von Wasser. Befriedigende Ergebnisse können aber auch erzielt werden, wenn geringe, den gewünschten Erfolg allenfalls unwesentlich beeinträchtigende Mengen an Wasser, beispielsweise bis zu 3 Gew.-% oder weniger, im Reaktionsgemisch vorhanden sind. Gegebenenfalls kann man Wasser bindende Mittel zufügen. Ein Wasser bindendes Mittel, das zudem katalytisch wirkt, ist Schwefelsäure.

Die Umsetzung (Umesterung und Decarboxylierung) läuft zwischen vielen Carbonsäuren und Keto-Ester-Verbindungen spontan mit mindestens befriedigender Geschwindigkeit ab; gegebenenfalls kann man die Umsetzung durch Erhitzen der Reaktionsmischung beschleunigen. Es wurde gefunden, daß Carbonsäuren mit höherer Säurestärke reaktiver sind. Ob die Umsetzung einer bestimmten Carbonsäure mit einer bestimmten Keto-Ester-Verbindung mit befriedigender Geschwindigkeit abläuft, kann der Fachmann einfach dadurch überprüfen, daß er die beiden Komponenten vermischt und langsam erwärmt. Die Freisetzung von Kohlendioxidgas zeigt die Umsetzung an. Durch elektronenziehende Gruppen aktivierte Carbonsäuren, beispielsweise Carbonsäuren, die in α-Stellung durch Halogen substituiert sind, reagieren sehr gut mit Keto-Ester-Verbindungen.

Stellt man fest, daß die Umsetzung bestimmter Carbonsäuren mit Keto-Ester-Verbindungen unerwünscht langsam abläuft, kann man die Reaktion katalysieren. Hierzu eignen sich Verbindungen mit größerer Säurestärke als die eingesetzte Carbonsäure, beispielsweise stärker saure Carbonsäuren wie Trifluoressigsäure, Mineralsäuren, wie Schwefelsäure oder Phosphorsäure, Sulfonsäuren oder auch "Onium"-Salze von Carbonsäuren. Selbstverständlich kann man eine derartige Katalyse auch für solche Umsetzungen vorsehen, die auch ohne zusätzlichen Katalysator in befriedigender Geschwindigkeit ablaufen, wie dies z.B. bei Trifluoressigsäure als eingesetzter Carbonsäure der Fall ist.

Eine bevorzugte Variante sieht vor, daß man einen Katalysator einsetzt, indem man die Decarboxylierung unter Umesterung mit einer Carbonsäure in Anwesenheit einer die Reaktion katalysierenden Menge eines "Onium"-Salzes einer Carbonsäure und/oder einer konzentrierten Säure ausgewählt aus der Gruppe umfassend Mineralsäuren, Arylsulfonsäuren und Alkylsulfonsäuren durchführt.

Der Begriff "Onium" steht für Kationen mit positiv geladenem Stickstoff, beispielsweise protonierte aromatische Stickstoffbasen wie Pyridinium oder protonierte Alkyl-, Dialkyl- oder Trialkylammonium-Kationen mit bis zu 20 C-Atomen, oder für durch Cycloalkyl substituierte Ammonium-Verbindungen oder cycloaliphatische Stickstoffbasen wie Piperidinium. Der Begriff "Mineralsäuren" umfaßt Oxosäuren von Elementen der 4. bis 7. Hauptgruppe, insbesondere Phosphorsäure, Schwefelsäure, Fluorsulfonsäure und Chlorsulfonsäure. Der Begriff "Alkylsulfonsäuren" umfaßt C1-C5-Alkylsulfonsäuren, wobei die Alkylgruppe durch 1 oder mehr Halogenatome substituiert sein kann, beispielsweise Methansulfonsäure und Trifluormethansulfonsäure. Der Begriff "Arylsulfonsäuren" umfaßt Phenylsulfonsäure sowie Sulfonsäuren mit einer Phenylgruppe, die durch 1 oder mehrere Halogenatome und/oder durch 1 oder mehrere C1-C2-Alkylgruppen substituiert sein kann, z. B. p.Toluolsulfonsäure. Die einzusetzende Menge an Carbonsäure beträgt vorteilhaft mindestens 0,8 Mol pro Mol umzuesternder Carboxy-Gruppe. Vorzugsweise arbeitet man wasserfrei.

Wenn n=1 ist, handelt es sich um Ketone der Formel $R^1C(O)CHR^2_2$. Hier können die beiden Substituenten $R^2$ gleiche oder unterschiedliche Bedeutung besitzen. Wenn n=2 ist, geht man aus von Dicarboxylat-Verbindungen der Formel $R^1C(O)CR^2[C(O)OR^4]_2$. Hier können die Substituenten $R^4$ gleiche oder unterschiedliche Bedeutung besitzen.

Ganz besonders bevorzugt führt man in der Variante der Erfindung die Umsetzung durch unter Decarboxylierung in Anwesenheit einer die Umesterung katalysierenden Menge eines "Onium"-Salzes einer Carbonsäure mit 1 bis 10 C-Atomen, eines Metall- oder "Onium"-Salzes einer durch mindestens 1 Halogenatom substituierten Carbonsäure mit 1 bis 10 C-Atomen, einer konzentrierten Mineralsäure, einer Arylsulfonsäure oder einer Alkylsulfonsäure.

Sofern man eine Carbonsäure in Anwesenheit eines "Onium"-Salzes einer Carbonsäure einsetzt, so kann es sich bei diesen um verschiedene Carbonsäuren oder vorzugsweise um gleiche Carbonsäuren handeln.

Als Mineralsäure setzt man vorzugsweise Schwefelsäure ein, die ganz besonders gut geeignet ist.

Hervorragend geeignet als Carbonsäuresalz sind "Onium"-Salze, wobei "Onium" für Kationen mit positiv geladenem Stickstoff steht, vorzugsweise für ein Kation des Stickstoffs der Formel $R^IR^{II}R^{III}R^{IV}N^+$, worin $R^I$, $R^{II}$, $R^{III}$ und $R^{IV}$ unabhängig voneinander Wasserstoff, Alkyl mit 1 bis 20 C-Atomen, oder Aralkyl stehen, oder worin $R^I$ und $R^{II}$ oder worin $R^{III}$ und $R^{IV}$, oder worin $R^I$, $R^{II}$ und $R^{III}$ oder worin $R^I$, $R^{II}$, $R^{III}$ und $R^{IV}$, gegebenenfalls unter Einschluß des Stickstoff-Atoms, gesättigte oder ungesättigte Ringsysteme bilden. Sehr gut geeignet sind Salze, in denen "Onium" für Ammonium, Pyridinium oder $R^{1'} R^{2'} R^{3'} R^{4'} N^+$ steht, worin $R^{1'}$, $R^{2'}$, $R^{3'}$ und $R^{4'}$ unabhängig voneinander Wasserstoff, Alkyl mit 1 bis 15 C-Atomen, Aryl oder Benzyl stehen. "Aryl" bedeutet hier insbesondere Phenyl oder durch 1 oder mehrere C1-C2-Alkylgruppen substituiertes Phenyl. Als Beispiel für solche Kationen seien genannt Pyridinium, Anilinium, Piperidinium, Benzyltriethylammonium und Triethylammonium.

Im erfindungsgemäßen Verfahren setzt man als Carbonsäure vorzugsweise eine Carbonsäure der Formel (III), $R^1COOH$, ein, worin $R^1$ die obenangegebene Bedeutung besitzt. Vorzugsweise steht $R^1$ für durch 1 bis 5 Halogenatome substituiertes Alkyl mit 1 oder 2 C-Atomen, insbesondere für $CH_2F$, $CHF_2$ oder $CF_3$. $R^2$ steht vorzugsweise für Wasserstoff, Fluor oder $C(O)R^3$, worin $R^3$ für $CH_3$, $CH_2F$, $CHF_2$ oder $CF_3$ steht.

Gemäß einer bevorzugten Ausführungsform steht $R^4$ für gegebenenfalls durch 1 oder mehr Halogenatome substituiertes Alkyl mit 1 bis 6 C-Atomen, vorzugsweise mit 1 bis 3 C-Atomen, insbesondere Methyl, Ethyl, Propyl, 1.1.1-Trifluorethyl oder Pentafluorpropyl.

Gemäß einer Ausführungsform der Erfindung bedeutet n=1. Man stellt dann Ketone der Formel $R^1C(O)CHR^2_2$ aus $R^1C(O)CR^2_2COOR^4$ her. Beispielsweise kann man $CF_3C(O)CH_3$ aus $CF_3C(O)CH_2COOMe$, $CF_3C(O)CH_2COOEt$ oder $CF_3C(O)CH_2COOCH_2CF_3$ herstellen. Analog kann man $CF_3C(O)CH_2F$ oder $CH_2FC(O)CH_2F$ und andere Ketone herstellen.

Gemäß einer anderen Ausführungsform ist n=2. Zur Herstellung von Ketonen der Formel $R^1C(O)CH_2R^2$ geht man dann aus von Dicarboxylaten der Formel $R^1C(O)CR^2[C(O)OR^4]_2$.

Zur Herstellung von Diketo-Verbindungen geht man aus von Keto-Estern der Formel $R^1C(O)CR^2[C(O)R^3][C(O)OR^4]$ oder $R^1C(O)C[C(O)R^3][C(O)OR^4]_2$.

Die Menge an Carbonsäure wird zweckmäßig so gewählt, daß pro Mol Estergruppe in der Verbindung der Formel (II) mindestens 0,8 Mol Carbonsäure eingesetzt werden. Vorteilhaft liegt das Molverhältnis von Carbonsäure zu Estergruppen in der Verbindung der Formel (II) zwischen 0,9:1 bis 1,1:1.

Die Temperatur bei der Verfahrensdurchführung liegt vorteilhaft im Bereich von 70 bis 130 °C. Der Druck liegt zweckmäßig im Bereich von 0,01 bar (absolut) bis 2 bar (absolut).

EP 0 623 575 B1

Das "Onium"-Salz der Carbonsäure bzw. die Mineralsäure liegt zweckmäßig in einer Konzentration von 50 bis 900 g/l in der Reaktionsmischung vor.

Gemäß einer anderen bevorzugten Variante setzt man als Carbonsäure die besonders starke Säure Trifluoressigsäure ein und verzichtet auf einen zusätzlichen Katalysator. Diese Variante des erfindungsgemäßen Verfahrens zur Herstellung von Ketonen durch Decarboxylierung von Keto-Ester-Verbindungen ist somit dadurch gekennzeichnet, daß man die Decarboxylierung unter Umesterung mit Trifluoressigsäure durchführt.

Katalysatoren wie "Onium"-Salze von Carbonsäuren oder Säuren wie Mineralsäuren, Arylsulfonsäuren und Alkylsulfonsäuren sind bei der Umesterung mit Trifluoressigsäure nicht notwendig. Die einzusetzende Menge an Trifluoressigsäure beträgt vorteilhaft mindestens 0,8 Mol pro Mol umzuesternder Carboxy-Gruppe. Vorzugsweise arbeitet man auch bei dieser Variante ohne Wasserzusatz, insbesondere wasserfrei und mit dem zu decarboxylierenden Ester als Edukt und Solvens.

Die Menge an Trifluoressigsäure wird zweckmäßig so gewählt, daß pro Mol Estergruppe in der Verbindung der Formel (II) mindestens 0,8 Mol Trifluoressigsäure eingesetzt werden. Vorteilhaft liegt das Molverhältnis von Trifluoressigsäure zu Estergruppen in der Verbindung der Formel (II) zwischen 0,9:1 bis 1,1:1.

Die Temperatur bei der Verfahrensdurchführung liegt auch hier vorteilhaft bei mindestens 70 °C, vorzugsweise im Bereich von 100 bis 130 °C. Der Druck liegt zweckmäßig im Bereich von 0,01 bar (absolut) bis 2 bar (absolut).

Gewünschtenfalls kann man im erfindungsgemäßen Verfahren in Anwesenheit eines inerten Lösungsmittels arbeiten, beispielsweise in Anwesenheit von Kohlenwasserstoffen oder perhalogenierten Verbindungen. Sofern flüssig, kann auch die verwendete Mineralsäure, die Carbonsäure oder der Keto-Ester als Lösungsmittel dienen. Bereits sehr geringe Mengen an Mineralsäuren wirken katalytisch. So reicht 1 Gew.-% oder weniger schon aus. Vorteilhaft setzt man 5 Gew.-% oder mehr zu.

Die als Ausgangsverbindungen dienenden Keto-Ester-Verbindungen sind bekannt oder können nach oder analog zu Standardmethoden hergestellt werden. Wie den Ausführungen weiter oben bereits entnommen werden kann, kann man mittels des erfindungsgemäßen Verfahrens auch β-Diketo-Verbindungen herstellen. Dazu geht man z. B. aus von Verbindungen der Formel (IV)a, $R^1C(O)CH_2C(O)OR^4$ oder (IV)b, $R^1C(O)CH[C(O)OR^4]_2$, oder (IV)c, $R^1C(O)CHR^2C(O)OR^4$ ($R^1$, $R^2$ und $R^4$ haben die oben beschriebene Bedeutung). Zur Herstellung dieser Verbindungen kann man das Proton gegen eine $R^3$ (CO)-Gruppe austauschen. Hierzu kann man beispielsweise die Verbindung der Formel (IV) mit $R^3C(O)Cl$ in Anwesenheit einer Base, z. B. Triethylamin, umsetzen. Beispielsweise kann man $CH_2(COOEt)_2$ mit $CF_3C(O)Cl$ und $Et_3N$ zu $CF_3C(O)CH(COOEt)_2$ umsetzen. Die Decarboxylierung des Keto-diesters mittels beispielsweise Trifluoressigsäure, gegebenenfalls in Anwesenheit von Schwefelsäure, liefert $CF_3C(O)CH_3$. Analog bildet sich aus $CH_3C(O)CH_2COOEt$ nach Trifluor-acetylierung und Decarboxylierung die Verbindung $CF_3C(O)CH_2C(O)CH_3$. Solche und ähnliche Diketone sind Komplexierungsmittel und z. B. beim Metallrecycling verwendbar.

Analog können auch andere Keto-Ester-Verbindungen durch Acylierung von Estern oder Diestern erzeugt werden.

Das erfindungsgemäße Verfahren kann auch kontinuierlich betrieben werden. Dazu leitet man in die Reaktionsmischung Carbonsäure und Keto-Ester-Verbindung ein und trennt gebildetes Keton, gegebenenfalls auch den gebildeten Ester, ab, beispielsweise durch Destillation.

Die vorliegende Erfindung weist - zusätzlich zur Ketonherstellung - noch einen weiteren Aspekt auf. Bei der Umesterung der Carbonsäure entsteht ein Ester dieser Carbonsäure, der aus dem Reaktionsgemisch isoliert werden kann. Durch Einsatz bestimmter Carbonsäuren und von Keto-Ester-Verbindungen mit bestimmten Ester-Gruppen können gezielt bestimmte, auf andere Weise eventuell nur schwer herstellbare Ester, beispielsweise der Trifluoressigsäure, synthetisiert werden.

Das erfindungsgemäße Verfahren gestaltet die technisch einfache Herstellung von Keto-Verbindungen, die wasserfrei anfallen. Daneben können gezielt auch Ester erzeugt werden. Die Auftrennung der Reaktionsgemische kann durch Destillation erfolgen.

Die Erfindung wird nun anhand von Beispielen weiter erläutert, ohne daß ihr Umfang eingeschränkt werden soll.

**Beispiel 1:**

Herstellung des als Ausgangsprodukt für die Decarboxylierung benötigten 1,1,1-Trifluor-2-oxo-3,3-diethyldicarboxylat durch Umsetzung von Trifluoracetylchlorid mit Malonsäurediethylester und Triethylamin

$$CF_3C(O)Cl + CH_2(COOEt)_2 \rightarrow CF_3C(O)CH(COOEt)_2$$

Die Herstellung erfolgte analog der von A.F. Ermolov, A.F. Eleev, A.F. Benda und G.A. Sokol'skii in Zh. Org. Khim. 23(1) (1987), Seiten 105-112 beschriebenen Methode. Dazu wurden annähernd äquimolare Mengen der Ausgangsverbindungen in Diethylether mit 2 Äquivalenten Triethylamin umgesetzt. Das gebildete $[CF_3C(O)C(COOEt)_2]^-HNEt_3^+$ wurde durch Zugabe von Schwefelsäure zu 1,1,1-Trifluor-2-oxo-3,3-diethyl-dicarboxylat umgesetzt. Diese Verbindung wurde

4

dann mit Diethylether aus dem Reaktionsgemisch extrahiert und destillativ gereinigt.

**Beispiel 2:**

Herstellung von 1,1,1-Trifluoraceton aus 1,1,1-Trifluor-2-oxo-3,3-diethyl-dicarboxylat durch eine Umesterung mit Trifluoressigsäure/Pyridiniumtrifluoracetat und anschließender Decarboxylierung

$$2CF_3CO_2H + CF_3C(O)CH(COOEt)_2 \xrightarrow[\text{Pyridinium} - \text{trifluoracetat}]{-2\ CO_2} CF_3C(O)CH_3 + 2\ CF_3CO_2Et$$

In einer Glasapparatur mit einer 20 cm Vigreuxkolonne mit zwei nachgeschalteten auf -78 °C gekühlten Kühlfallen und KPG-Rührer wurden 35,9 g (0,45 mol) Pyridin vorgelegt und 149,2 g (1,30 mol) Trifluoressigsäure unter leichter Kühlung bei 50 bis 70 °C zugetropft. Nach der Zugabe von 51,2 g (0,20 mol) 1,1,1-Trifluor-2-oxo-3,3-diethyl-dicarboxylat wurde die Reaktionsmischung im Ölbad auf 120 bis 140 °C erhitzt, wobei bei 90 °C die Decarboxylierung einsetzte. Die Ausbeute an 1,1,1-Trifluoraceton betrug nach einer Reaktionszeit von 10 h 72,6 % der Theorie als Mischung mit Trifluoressigsäureethylester.

**Beispiel 3:**

Herstellung von 1,1,1-Trifluoraceton aus $\omega,\omega,\omega$-Trifluoracetessigsäureethylester durch Umesterung mit Trifluoressigsäure in konzentrierter Schwefelsäure und Decarboxylierung bei 90 bis 120 °C

$$CF_3CO_2H + CF_3C(O)CH_2C(O)OEt \xrightarrow[-CO_2]{\text{conc. } H_2SO_4} CF_3C(O)CH_3 + CF_3CO_2Et$$

In einer Glasapparatur mit einer 20 cm Vigreuxkolonne mit zwei nachgeschalteten auf -78 °C gekühlten Kühlfallen und KPG-Rührer wurden 100 ml conz. $H_2SO_4$ (dient auch als Solvens) und 109,5 g (0,96 mol) Trifluoressigsäure bei Raumtemperatur vorgelegt. Nach der Zugabe von 58,9 g (0,32 mol) $\omega,\omega,\omega$-Trifluoracetessigsäureethylester wurde die Reaktionsmischung unter Rühren im Ölbad auf Temperaturen von 90 bis 120 °C Innentemperatur erhitzt, wobei bei ca. 75 °C die Decarboxylierung einsetzte. Zu Reaktionsbeginn wurde die überschüssige Trifluoressigsäure teilweise abdestilliert. Die Ausbeute an 1,1,1-Trifluoraceton betrug nach einer Reaktionszeit von 7 h 86 % d. Theorie als Mischung mit Trifluoressigsäureethylester, wobei zum abschließenden Ausheizen des Trifluoressigsäureethylesters und noch vorhandener Trifluoressigsäure die Ölbadtemperatur bis auf 160 °C gesteigert wurde. Anschließende Destillation über eine Füllkörperkolonne ergab reines Trifluoraceton.

**Beispiel 4:**

Herstellung von 1,1,1-Trifluoraceton aus 1,1,1-Trifluor-2-oxo-3,3-diethyl-dicarboxylat durch Umesterung mit Trifluoressigsäure/Methansulfonsäure und anschließender Decarboxylierung bei 130 bis 160 °C

$$2CF_3CO_2H + CF_3C(O)CH(COOEt)_2 \xrightarrow[-2\ CO_2]{CH_3SO_3H} CF_3C(O)CH_3 + 2\ CF_3CO_2Et$$

In einer Glasapparatur mit einer 20 cm Vigreuxkolonne mit zwei nachgeschalteten auf -78 °C gekühlten Kühlfallen und KPG-Rührer wurden 148,24 g (1,54 mol) Methansulfonsäure und 68,41 g (0,60 mol) Trifluoressigsäure bei Raumtemperatur vorgelegt. Nach der Zugabe von 51,24 g (0,20 mol) 1,1,1-Trifluor-2-oxo-3,3-diethyl-dicarboxylat wurde die Reaktionsmischung unter Rühren (600 U/min) im Ölbad auf Temperaturen von 130 bis 160 °C Innentemperatur erhitzt, wobei bei ca. 90 °C die Decarboxylierung einsetzte. Zu Reaktionsbeginn wurde die überschüssige Trifluoressigsäure teilweise abdestilliert. Die Ausbeute an 1,1,1-Trifluoraceton betrug nach einer Reaktionszeit von 9 h 79 % d. Theorie als Mischung mit Trifluoressigsäureethylester, wobei zum abschließenden Ausheizen des Trifluoressigsäureethylesters und der überschüssigen Trifluoressigsäure die Ölbadtemperatur bis auf 200 °C gesteigert wurde.

**Beispiel 5:**

Herstellung von 1,1,1-Trifluoraceton aus 1,1,1-Trifluor-2-oxo-3,3-Diethyl-dicarboxylat durch Umesterung mit Trifluoressigsäure in konzentrierter Schwefelsäure und Decarboxylierung bei 90 bis 120 °C

$$2CF_3CO_2H + CF_3C(O)CH(COOEt)_2 \xrightarrow[-2\ CO_2]{\text{conc. } H_2SO_4} CF_3C(O)CH_3 + 2CF_3CO_2Et$$

In einer Glasapparatur mit einer 20 cm Vigreuxkolonne mit zwei nachgeschalteten auf -78 °C gekühlten Kühlfallen und KPG-Rührer wurden 100 ml conz. $H_2SO_4$ und 68,41 g (0,60 mol) Trifluoressigsäure bei Raumtemperatur vorgelegt.

Nach der Zugabe von 51,24 g (0,20 mol) 1,1,1-Trifluor-2-oxo-3,3-diethyl-dicarboxylat wurde die Reaktionsmischung unter Rühren (600 U/min) im Ölbad auf Temperaturen von 90 bis 120 °C Innentemperatur erhitzt, wobei bei ca 90 °C die Decarboxylierung einsetzte. Zu Reaktionsbeginn wurde die überschüssige Trifluoressigsäure teilweise abdestilliert. Die Ausbeute an 1,1,1-Trifluoraceton betrug nach einer Reaktionszeit von 7 h 82 % d. Theorie als Mischung mit Trifluoressigsäureethylester, wobei zum abschließenden Ausheizen des Trifluoressigsäureethylesters und noch vorhandener Trifluoressigsäure die Ölbadtemperatur bis auf 160 °C gesteigert wurde.

Auch eine Schwefelsäure/Sulfolan-Mischung (30 ml conc. Schwefelsäure/70 ml Sulfolan) wurde getestet und lieferte annähernd gleiche Ergebnisse. Die Durchführung mehrerer Decarboxylierungsdurchgänge mit derselben Katalysatormischung war ohne Probleme möglich. Beim Einsatz von Chlor- und Fluorsulfonsäure als Katalysatorkomponente kam es zur Bildung von nicht identifizierten Nebenkomponenten. Wird zu Reaktionsbeginn zu viel Trifluoressigsäure abgenommen, erfolgt die Decarboxylierung unter Bildung von Ethylen.

Die Trennung des aus allen Reaktionen erhaltenen Trifluoraceton/Trifluoressigsäureethylestergemisches erfolgte anschließend durch Destillation über eine 40-cm-Füllkörperkolonne.

### Beispiel 6:

Kontinuierliche Herstellung von 1,1,1-Trifluoraceton in einem Dauerversuch

Beispiel 3 wurde wiederholt. Entsprechend der Menge an 1,1,1-Trifluoraceton und Trifluoressigsäureethylester, die aus dem Reaktionsgemisch abdestilliert wurden, wurde kontinuierlich Edukt in die Glasapparatur eingeleitet. Die Ausbeute an 1,1,1-Trifluoraceton nach einer Versuchsdauer von 15 Tagen betrug 95 % d. Theorie.

### Beispiel 7:

Verfahren zur kontinuierlichen Herstellung von 1.1.1-Trifluoraceton durch Umesterung von $\omega$, $\omega$, $\omega$-Trifluoracetessigsäureethylester mit Trifluoressigsäure unter decarboxylierenden Bedingungen ohne Zusatz einer katalysierenden Mineralsäure

$$CF_3CO_2H + CF_3C(O)CH_2C(O)OEt \xrightarrow{-CO_2} CF_3C(O)CH_3 + CF_3CO_2Et$$

In einem 250 ml Dreihalskolben mit mechanischem Rührer, 20 cm-Vigreuxkolonne und zwei nachgeschalteten, auf -78 °C gekühlten Kühlfallen wurden 149,5 g (0,81 mol) Trifluoracetessigsäureethylester und 30,8 g (0,27 mol) Trifluoressigsäure vorgelegt. Die Mischung wurde im Ölbad auf 120 °C erhitzt, wobei die Decarboxylierung bei 70 °C begann. Die kontinuierliche Betriebsweise wurde durch Konstanthalten des Füllstandes und durch Zutropfen eines äquimolaren Gemisches aus den beiden Reaktionskomponenten erreicht. Während einer Reaktionszeit von 17,2 h wurden ingesamt 1,08 mol Trifluoracetessigsäureethylester zu Trifluoraceton umgesetzt. Die Gesamtausbeute an Trifluoraceton betrug 96,9 %, die an Trifluroessigsäureethylester 99 %. Die Trennung des Trifluoressigsäureethylester/Trifluoraceton-Gemisches erfolgte durch Destillation über eine 40 cm-Füllkörperkolonne.

### Beispiel 8:

Verfahren zur kontinuierlichen Darstellung von 1,1,1-Trifluoraceton durch Umesterung von $\omega,\omega,\omega$-Trifluoracetessigsäureethylester mit Trifluoressigsäure unter decarboxylierenden Bedingungen mit $\omega,\omega,\omega$-Trifluoracetessigsäureethylester als Reaktionskomponente ($H_2SO_4$-Katalyse mit verringerter $H_2SO_4$-Menge)

In einem 250 ml Dreihalskolben mit KPG-Rührer, 20 cm Vigreuxkolonne und zwei nachgeschalteten, auf -78 °C gekühlten Kühlfallen wurden 151,0 g (0,82 mol) Trifluoracetessigsäureethylester und 30,8 g (0,27 mol) Trifluoressigsäure vorgelegt und mit 20 ml conc. $H_2SO_4$ versetzt. Die Mischung wurde im Ölbad auf 110 °C (Sumpftemperatur) erhitzt, wobei die Decarboxylierung bei 75 °C langsam einsetzte. Die kontinuierliche Betriebsweise wurde durch Konstanthalten des Füllstandes, durch Zutropfen eines äquimolaren Gemisches aus den beiden Reaktionskomponenten erreicht. Umgesetzt wurde stündlich nach dieser Betriebsweise 159 mmol Trifluoracetessigsäurethylester-/Trifluoressigsäure-Gemisch zu Trifluoraceton und Trifluoressigsäureethylester. Die Gesamtausbeute an isoliertem Trifluoraceton betrug 92,9 %, die an Trifluoressigsäureethylester 99 % (als Gemisch). Die Trennung des Gemisches erfolgte durch Destillation über eine 40 cm Füllkörperkolonne.

### Patentansprüche

1. Verfahren zur Herstellung von Ketonen aus Keto-Ester-Verbindungen, wobei man die Keto-Ester-Verbindung mit einer Carbonsäure umestert und die freigesetzte Säure decarboxyliert, um das Keton zu erhalten und man Ketone der allgemeinen Formel (I) herstellt

$$R^1C(O)CH_nR^2_{3-n} \qquad\qquad (I)$$

worin $R^1$ Alkyl mit 1 bis 10 C-Atomen; durch mindestens 1 Halogenatom substituiertes Alkyl mit 1 bis 10 C-Atomen; Aryl; durch mindestens 1 Halogenatom substituiertes Aryl; Arylalkyl bedeutet; $R^2$ für Wasserstoff, Alkyl mit 1 bis 10 C-Atomen; Aryl; durch mindestens 1 Halogenatom substituiertes $C_1$-$C_{10}$-alkyl; durch mindestens 1 Halogenatom substituiertes Aryl; Arylalkyl, Halogen oder $C(O)R^3$, worin $R^3$ Alkyl mit 1 bis 10 C-Atomen; durch mindestens 1 Halogenatom substituiertes Alkyl mit 1 bis 10 C-Atomen; Aryl; durch mindestens 1 Halogenatom substituiertes Aryl; Arylalkyl bedeutet und n = 1 oder 2 bedeutet, durch Umsetzung einer Carbonsäure mit 1 bis 10 C-Atomen oder einer durch mindestens 1 Halogenatom substituierten Carbonsäure mit 1 bis 10 C-Atomen bei einer Temperatur von mindestens 70 °C mit einer Keto-Ester-Verbindung in Form einer Carboxylat- oder Dicarboxylatverbindung der allgemeinen Formel (II)

$$R^1C(O)CR^2_{3-n}[C(O)OR^4]_n \qquad\qquad (II)$$

worin n, $R^1$ und $R^2$ die obenbezeichnete Bedeutung besitzen; $R^4$ Alkyl mit 1 bis 10 C-Atomen; durch mindestens 1 Halogenatom substituiertes Alkyl mit 1 bis 10 C-Atomen; Arylalkyl; Aryl; durch mindestens 1 Halogenatom substituiertes Aryl, bedeutet.

2. Verfahren nach Anspruch 1, dadurch gekennzeichnet, daß man als Carbonsäure eine Carbonsäure der Formel (III), $R^1COOH$, einsetzt, worin $R^1$ die obengenannte Bedeutung besitzt.

3. Verfahren nach einem der vorhergehenden Ansprüche, dadurch gekennzeichnet, daß $R^1$ für durch 1 bis 5 Halogenatome substituiertes Alkyl mit 1 oder 2 C-Atomen steht.

4. Verfahren nach Anspruch 3, dadurch gekennzeichnet, daß $R^1$ für $CH_2F$, $CHF_2$ oder $CF_3$ steht.

5. Verfahren nach einem der vorhergehenden Ansprüche, dadurch gekennzeichnet, daß $R^2$ für Wasserstoff, Fluor oder $C(O)R^3$ steht, worin $R^3$ $CH_3$, $CH_2F$, $CHF_2$ oder $CF_3$ bedeutet.

6. Verfahren nach einem der vorhergehenden Ansprüche, dadurch gekennzeichnet, daß $R^4$ gegebenenfalls durch Halogen substituiertes Alkyl mit 1 bis 6 C-Atomen, vorzugsweise mit 1 bis 3 C-Atomen bedeutet.

7. Verfahren nach einem der vorhergehenden Ansprüche, dadurch gekennzeichnet, daß $R^4$ für Methyl, Ethyl, Propyl, 1,1,1-Trifluorethyl oder Pentafluorpropyl steht.

8. Verfahren nach einem der Ansprüche 1 bis 7, dadurch gekennzeichnet, daß man die Decarboxylierung unter Umesterung mit einer Carbonsäure in Anwesenheit einer die Umesterung katalysierenden Menge eines "Onium"-Salzes einer Carbonsäure und/oder einer konzentrierten Säure ausgewählt aus der Gruppe umfassend Mineralsäuren, Arylsulfonsäuren und Alkylsulfonsäuren durchführt.

9. Verfahren nach Anspruch 8, dadurch gekennzeichnet, daß man die Decarboxylierung unter Umesterung in Anwesenheit einer die Umesterung katalysierenden Menge eines "Onium"-Salzes einer Carbonsäure mit 1 bis 10 C-Atomen, eines Metall- oder "Onium"-Salzes einer durch mindestens 1 Halogenatom substituierten Carbonsäure mit 1 bis 10 C-Atomen, einer konzentrierten Mineralsäure, Arylsulfonsäure, einer Alkylsulfonsäure oder einer durch mindestens 1 Halogenatom substituierten Carbonsäure mit bis zu 4 C-Atomen durchführt.

10. Verfahren nach Anspruch 1, dadurch gekennzeichnet, daß man die Umsetzung der Verbindung der Formel (II) mit der Carbonsäure in Anwesenheit einer die Umesterung katalysierenden Menge eines "Onium"-Salzes der gleichen Carbonsäure oder einer konzentrierten Mineralsäure ausgewählt aus der Schwefelsäure und Phosphorsäure umfassenden Gruppe oder Methansulfonsäure durchführt.

11. Verfahren nach Anspruch 10, dadurch gekennzeichnet, daß man ein "Onium"-Salz der Carbonsäure oder Schwefelsäure einsetzt.

12. Verfahren nach einem der vorhergehenden Ansprüche, dadurch gekennzeichnet, daß man zur Herstellung von $CF_3C(O)CH_3$ Trifluoressigsäure umsetzt mit $CF_3C(O)CH_2COOEt$ oder $CF_3C(O)CH(COOEt)_2$ in Anwesenheit einer die Umesterung katalysierenden Menge eines "Onium"-Salzes der Trifluoressigsäure oder konzentrierter Schwefelsäure.

**13.** Verfahren nach einem der vorhergehenden Ansprüche, dadurch gekennzeichnet, daß das "Onium"-Salz der Carbonsäure bzw. die konzentrierte Mineralsäure in einer Konzentration von 50 bis 900 g/l in der Reaktionsmischung vorhanden ist.

**14.** Verfahren nach einem der Ansprüche 1 bis 7, dadurch gekennzeichnet, daß man als Carbonsäure Trifluoressigsäure einsetzt in Abwesenheit eines zusätzlichen Katalysators.

**15.** Verfahren nach Anspruch 14, dadurch gekennzeichnet, daß man zur Herstellung von $CF_3C(O)CH_3$ Trifluoressigsäure umsetzt mit $CF_3C(O)CH_2COOEt$ oder $CF_3C(O)CH(COOEt)_2$.

**16.** Verfahren nach einem der vorhergehenden Ansprüche, dadurch gekennzeichnet, daß pro Estergruppe 0,9 bis 1,1 Mol Trifluoressigsäure eingesetzt werden.

**17.** Verfahren nach einem der vorhergehenden Ansprüche, dadurch gekennzeichnet, daß bei einer Temperatur zwischen 100 °C und 130 °C, umgeestert wird.

**18.** Abänderung des Verfahrens nach einem der Ansprüche 1 bis 17, dadurch gekennzeichnet, daß man zur kontinuierlichen Durchführung unter Abtrennung des gebildeten Ketons und des gegebenenfalls gebildeten Esters, in die Reaktionsmischung die Carbonsäure und die Keto-Ester-Verbindung einleitet.

**Claims**

**1.** Process for preparing ketones from ketoester compounds, in which the ketoester compound is transesterified with a carboxylic acid and the acid liberated is decarboxylated to obtain the ketone, and ketones of the general formula (I)

$$R^1C(O)CH_nR^2_{3-n} \qquad (I)$$

are prepared in which $R^1$ is alkyl having from 1 to 10 carbon atoms; alkyl substituted by at least 1 halogen atom and having from 1 to 10 carbon atoms; aryl; aryl substituted by at least 1 halogen atom; arylalkyl; $R^2$ is hydrogen, alkyl having from 1 to 10 carbon atoms; aryl; $C_1$-$C_{10}$-alkyl substituted by at least one halogen atom; aryl substituted by at least 1 halogen atom; arylalkyl, halogen or $C(O)R^3$, in which $R^3$ is alkyl having from 1 to 10 carbon atoms; alkyl substituted by at least 1 halogen atom and having from 1 to 10 carbon atoms; aryl; aryl substituted by at least 1 halogen atom; or arylalkyl, and n = 1 or 2, by reaction of a carboxylic acid having from 1 to 10 carbon atoms or a carboxylic acid substituted by at least 1 halogen atom and having from 1 to 10 carbon atoms at a temperature of at least 70°C with a ketoester compound in the form of a carboxylate or dicarboxylate compound of the general formula (II)

$$R^1C(O)CR^2_{3-n}[C(O)OR^4]_n \qquad (II)$$

in which n, $R^1$ and $R^2$ have the meanings given above; $R^4$ is alkyl having from 1 to 10 carbon atoms; alkyl substituted by at least 1 halogen atom and having from 1 to 10 carbon atoms; arylalkyl; aryl; or aryl substituted by at least 1 halogen atom.

**2.** Process according to Claim 1, characterized in that the carboxylic acid used is a carboxylic acid of the formula (III), $R^1COOH$, in which $R^1$ possesses the meaning given above.

**3.** Process according to one of the preceding claims, characterized in that $R^1$ is alkyl substituted by from 1 to 5 halogen atoms and having 1 or 2 carbon atoms.

**4.** Process according to Claim 3, characterized in that $R^1$ is $CH_2F$, $CHF_2$ or $CF_3$.

**5.** Process according to one of the preceding claims, characterized in that $R^2$ is hydrogen, fluorine or $C(O)R^3$ in which $R^3$ is $CH_3$, $CH_2F$, $CHF_2$ or $CF_3$.

**6.** Process according to one of the preceding claims, characterized in that $R^4$ is alkyl which is optionally substituted by halogen and has from 1 to 6 carbon atoms, preferably from 1 to 3 carbon atom.

7. Process according to one of the preceding claims, characterized in that $R^4$ is methyl, ethyl, propyl, 1,1,1-trifluoroethyl or pentafluoropropyl.

8. Process according to one of Claims 1 to 7, characterized in that the decarboxylation is carried out under transesterification with a carboxylic acid in the presence of a transesterification-catalyzing amount of an "onium" salt of a carboxylic acid and/or of a concentrated acid selected from the group comprising mineral acids, arylsulphonic acids and alkylsulphonic acids.

9. Process according to Claim 8, characterized in that the decarboxylation is carried out under transesterification in the presence of a transesterification-catalyzing amount of an "onium" salt of a carboxylic acid having from 1 to 10 carbon atoms, of a metal or "onium" salt of a carboxylic acid substituted by at least 1 halogen atom and having from 1 to 10 carbon atoms, of a concentrated mineral acid, arylsulphonic acid, of an alkylsulphonic acid or of a carboxylic acid substituted by at least 1 halogen atom and having up to 4 carbon atoms.

10. Process according to Claim 1, characterized in that the reaction of the compound of the formula (II) with the carboxylic acid is carried out in the presence of a transesterification-catalyzing amount of an "onium" salt of the same carboxylic acid or of a concentrated mineral acid selected from the group comprising sulphuric acid and phosphoric acid or methanesulphonic acid.

11. Process according to Claim 10, characterized in that an "onium" salt of the carboxylic acid or sulphuric acid is used.

12. Process according to one of the preceding claims, characterized in that $CF_3C(O)CH_3$ is prepared by reacting trifluoroacetic acid with $CF_3C(O)CH_2COOEt$ or $CF_3C(O)CH(COOEt)_2$ in the presence of a transesterification-catalyzing amount of an "onium" salt of trifluoroacetic acid or concentrated sulphuric acid.

13. Process according to one of the preceding claims, characterized in that the "onium" salt of the carboxylic acid or the concentrated mineral acid is present in a concentration from 50 to 900 g/l in the reaction mixture.

14. Process according to one of Claims 1 to 7, characterized in that trifluoroacetic acid is used as carboxylic acid in the absence of an additional catalyst.

15. Process according to Claim 14, characterized in that $CF_3C(O)CH_3$ is prepared by reacting trifluoroacetic acid with $CF_3C(O)CH_2COOEt$ or $CF_3C(O)CH(COOEt)_2$.

16. Process according to one of the preceding claims, characterized in that from 0.9 to 1.1 mol of trifluoroacetic acid are used per ester group.

17. Process according to one of the preceding claims, characterized in that the transesterification is carried out at a temperature of between 100°C and 130°C.

18. Modification of the process according to one of Claims 1 to 17, characterized in that, for continuous execution with removal of the ketone formed and the ester optionally formed, the carboxylic acid and the ketoester compound are fed into the reaction mixture.

## Revendications

1. Procédé de préparation de cétones à partir de composé céto-esters, dans lequel on transestérifie le composé céto-ester avec un acide carboxylique et on décarboxyle l'acide libéré pour obtenir la cétone et on prépare des cétones de formule générale (I)

$$R^1C(O)CH_nR^2_{3-n} \qquad \qquad (I)$$

dans laquelle $R^1$ représente un alkyle en $C_1$ à $C_{10}$ ; un alkyle en $C_1$ à $C_{10}$ substitué par au moins un atome d'halogène ; un aryle ; un aryle substitué par au moins un atome d'halogène ; un arylalkyle ; $R^2$ représente un hydrogène, un alkyle en $C_1$ à $C_{10}$ ; un aryle ; un alkyle en $C_1$ à $C_{10}$ qui est substitué par au moins un atome d'halogène ; un aryle substitué par au moins un atome d'halogène ; un arylalkyle ; un halogène ou $C(O)R^3$, où $R^3$ représente un alkyle en $C_1$ à $C_{10}$ ; un alkyle en $C_1$ à $C_{10}$ substitué par au moins un atome d'halogène ; un aryle ; un aryle substitué par au moins un atome d'halogène ; un arylalkyle et n = 1 ou 2, par réaction d'un acide carboxylique avec de 1 à 10 atomes de carbone ou d'un acide carboxylique en $C_1$ à $C_{10}$ substitué par au moins un atome d'halogène, à une température d'au moins 70°C, avec un composé céto-ester sous la forme d'un composé carboxylate ou dicarboxylate de formule générale (II)

$$R^1C(O)CR^2{}_{3-n}[C(O)OR^4]_n \qquad\qquad (II)$$

dans laquelle n, $R^1$ et $R^2$ ont la signification donnée ci-dessus et $R^4$ représente un alkyle en $C_1$ à $C_{10}$ ; un alkyle en $C_1$ à $C_{10}$ substitué par au moins un atome d'halogène ; un arylalkyle ; un aryle ; un aryle substitué par au moins un atome d'halogène.

2. Procédé selon la revendication 1, caractérisé en ce qu'on utilise comme acide carboxylique un acide carboxylique de formule (III), $R^1COOH$, dans laquelle $R^1$ a la signification indiquée ci-dessus.

3. Procédé selon l'une des revendications précédentes caractérisé en ce que $R^1$ représente un alkyle en $C_1$ à $C_2$ substitué par de 1 à 5 atomes d'halogène.

4. Procédé selon la revendication 3, caractérisé en ce que $R^1$ représente $CH_2F$, $CHF_2$ ou $CF_3$.

5. Procédé selon l'une des revendications précédentes, caractérisé en ce que $R^2$ représente un hydrogène, un fluor ou $C(O)R^3$, où $R^3$ représente $CH_3$, $CH_2F$, $CHF_2$ ou $CF_3$.

6. Procédé selon l'une des revendications précédentes, caractérisé en ce que $R^4$ représente un alkyle en $C_1$ à $C_6$, de préférence en $C_1$ à $C_3$. éventuellement substitué par un halogène.

7. Procédé selon l'une des revendications précédentes, caractérisé en ce que $R^4$ représente un méthyle, éthyle, propyle, 1,1,1-trifluoroéthyle ou pentafluoropropyle.

8. Procédé selon l'une des revendications 1 à 7, caractérisé en ce qu'on procède à la décarboxylation avec transestérification au moyen d'un acide carboxylique en présence d'une quantité d'un sel d'"onium" d'un acide carboxylique et/ou d'un acide concentré du groupe comprenant les acides minéraux, les acides aryl sulfoniques et les acides alkyl sulfoniques, catalysant la transestérification.

9. Procédé selon la revendication 8, caractérisé en ce qu'on conduit la décarboxylation avec transestérification en présence d'une quantité d'un sel d'"onium" d'un acide carboxylique en $C_1$ à $C_{10}$, d'un sel métallique ou d'"onium", d'un acide carboxylique en $C_1$ à $C_{10}$ substitué par au moins un atome d'halogène, d'un acide minéral concentré, d'un acide aryl sulfonique, d'un acide alkyl sulfonique ou d'un acide carboxylique ayant jusqu'à 4 atomes de carbone substitué par au moins un atome d'halogène, catalysant la transestérification.

10. Procédé selon la revendication 1, caractérisé en ce qu'on conduit la transestérification du composé de formule (II) avec l'acide carboxylique en présence d'une quantité d'un sel d'"onium" du même acide carboxylique ou d'un acide minéral concentré choisi dans le groupe comprenant l'acide sulfurique et l'acide phosphorique ou l'acide méthane sulfonique, catalysant la trans-estérification.

11. Procédé selon la revendication 10, caractérisé en ce qu'on utilise un sel d'"onium" de l'acide carboxylique ou de l'acide sulfurique.

12. Procédé selon une des revendications précédentes, caractérisé en ce que pour préparer $CF_3C(O)CH_3$ on fait réagir de l'acide trifluoro-acétique avec $CF_3C(O)CH_2COOEt$ ou $CF_3C(O)CH(COOEt)_2$ en présence d'une quantité d'un sel d'"onium" de l'acide trifluoro-acétique ou de l'acide sulfurique concentré catalysant la transestérification.

13. Procédé selon l'une des revendications prédécentes, caractérisé en ce que le sel d'"onium" de l'acide carboxylique ou l'acide minéral concentré est présent dans le mélange réactionnel à une concentration de 50 à 900 g/litre.

14. Procédé selon l'une des revendications 1 à 7, caractérisé en ce qu'on utilise comme acide carboxylique l'acide trifluoro-acétique en l'absence d'un catalyseur supplémentaire.

15. Procédé selon la revendication 14, caractérisé en ce que pour préparer $CF_3C(O)CH_3$ on fait réagir de l'acide trifluoro-acétique avec $CF_3C(O)CH_2COOEt$ ou $CF_3C(O)CH(COOEt)_2$.

**16.** Procédé selon l'une des revendications précédentes, caractérisé en ce qu'on utilise par groupe ester de 0,9 à 1,1 mole d'acide trifluoro-acétique.

**17.** Procédé selon l'une des revendications précédentes, caractérisé en ce qu'on procède à la transestérification à une température comprise entre 100°C et 130°C.

**18.** Modification du procédé selon l'une des revendications 1 à 17 caractérisée en ce que pour la réalisation continue avec séparation de l'acétone formée et de l'ester éventuellement formé on introduit dans le mélange réactionnel l'acide carboxylique et le composé céto-ester.